# EUROPÄISCHE PATENTSCHRIFT

(11) **EP 4 045 674 B1**
(45) Veröffentlichungstag und Bekanntmachung des Hinweises auf die Patenterteilung: **20.08.2025**
(21) Anmeldenummer: 20792644.5
(22) Anmeldetag: 16.10.2020
(51) Int. Cl.: C07K 1/14, C12N 9/40, C12P 21/00, C12P 21/02

(54) **VERFAHREN ZUR GEWINNUNG VON MATERIAL VON PFLANZENZELLOBERFLÄCHEN**
METHOD FOR OBTAINING MATERIAL FROM PLANT CELL SURFACES
PROCÉDÉ D'OBTENTION DE MATÉRIAU DES SURFACES DE CELLULES VÉGÉTALES

(30) Priorität: 17.10.2019 EP 19203842
(43) Veröffentlichungstag der Anmeldung: 24.08.2022
(73) Patentinhaber: eleva GmbH, 79108 Freiburg (DE)
(72) Erfinder: JAHN, Berengar, 76646 Bruchsal (AT); NIEDERKRÜGER, Holger, 79364 Malterdingen (DE); KOCH, Jonas, 79117 Freiburg (DE); BUSCH, Andreas, 79285 Ebringen (DE); SCHAAF, Andreas, 79104 Freiburg (DE)
(74) Vertreter: SONN Patentanwälte GmbH & Co KG
(86) Internationale Anmeldenummer: PCT/EP2020/079143
(87) Internationale Veröffentlichungsnummer: WO 2021/074354

(56) Entgegenhaltungen:
- WO-A1-2012/126123
- WO-A1-2017/030592
- WO-A2-00/09725
- DE-A1- 102005 031 459
- US-A1- 2015 140 644
- EVA L DECKER ET AL: "Current achievements in the production of complex biopharmaceuticals with moss bioreactors", BIOPROCESS AND BIOSYSTEMS ENGINEERING, SPRINGER, BERLIN, DE, vol. 31, no. 1, 14 August 2007 (2007-08-14), pages 3 - 9, XP019564175, ISSN: 1615-7605

## Beschreibung

### Gebiet der Erfindung

Die vorliegende Erfindung betrifft die Isolierung von Proteinen aus Zellen.

### Hintergrund der Erfindung

Verfahren zur Isolierung von Proteinen aus Zellen oder Zellverbänden beinhalten die osmotische Lyse, enzymatische oder chemische Lyse, Ultraschallbehandlung und mechanischen Aufschluss. Zum mechanischen Aufschluss werden in der Regel Zellen mit einem Homogenisator oder Mixer zerkleinert.

Kurze Behandlungszeiten können auch verwendet werden, um Zellverbände zu teilen und um Zellen in Suspension zu bringen, wobei es nur zu einer teilweisen Lyse der gesamten Zellemenge kommt (Orellana-Escobedo et al., Plant Cell Rep. 2015, 34 (3) : 425-33) .

Sofern ein Protein aus einzelnen Organellen oder Zellkompartments isoliert wird, werden üblicherweise vor einem Aufschluss die Zellorganellen isoliert und dann vom Isolat ein Aufschluss vorgenommen.

Witzel et al., Plant Methods 2011, 7:48; Leary et al., J Vis Exp. 2014; (94): 52113; und Córdoba-Pedregosa et al., Plant Physiology 1996, 112(3):1119-1125, beschreiben Verfahren um Proteine aus dem Apoplasten von Pflanzenzellen zu extrahieren. Als Apoplast wird der Raum außerhalb der Protoplasten bezeichnet. Er besteht aus den Zellwänden und dem Interzellularraum. Die in diesen Publikationen beschriebenen Verfahren beinhalten eine osmotische Extraktion mit unterschiedlichen Infiltrationslösungen (z.B. Salze) und Zentrifugation. Dieses Verfahren hat allerdings den Nachteil, dass bei dieser Extraktion nur die durch Infiltration zugänglichen Materialien extrahiert werden können.

US 2015/0140644 A1 und AU 2017202473 B2 beschreiben Verfahren zum Gewinnen von Proteinen aus dem Apoplasten mit den Schritten der Lyse der Zellwand, einer Inkubation und Extraktion. Hierbei sind enzymatische oder chemische Modifikationen der Proteine möglich.

Es ist ein Ziel der Erfindung, verbesserte Isolier- oder Extraktionsmöglichkeiten von Materialien des Apoplasten, insbesondere von in den Apoplasten sekretiertem Material, zur Verfügung zu stellen.

### Zusammenfassung der Erfindung

Die vorliegende Erfindung betrifft ein Verfahren zum Ablösen von exprimiertem Material von der Oberfläche oder aus dem Apoplast von Pflanzenzellen, wobei die Pflanzenzellen in einem flüssigen Medium mit einem Rotor-Stator behandelt werden, wobei die durch Rotation des Rotors eingebrachte spezifische Wärme des Rotor-Stators maximal 3 kJ pro kg des flüssigen Mediums und pro g/l Trockenmasse der Pflanzenzellen und die eingebrachte spezifische Wärmeleistung in das Medium maximal 1,5 kJ pro kg des flüssigen Mediums pro Minute und pro g/l Trockenmasse der Pflanzenzellen beträgt.

Ebenso betrifft die Erfindung in einem weiteren Aspekt ein Verfahren zum Ablösen von exprimiertem Material von der Oberfläche oder dem Apoplast einer oder mehrerer Pflanzenzellen, wobei die eine oder mehrere Pflanzenzelle in einem flüssigen Medium mit einem Rotor-Stator behandelt wird, wobei die durch Rotation des Rotors eingebrachte Wärme des Rotor-Stators maximal 30 kJ pro kg des flüssigen Mediums und die eingebrachte Wärmeleistung in das Medium maximal 1,5 kJ pro kg des flüssigen Mediums und pro Minute beträgt.

Die Parameter beider Aspekte können kombiniert werden, insbesondere da es sich bloß um andere Bezugsgrößen handelt, wobei das Wesen der Erfindung gleichsam erfüllt wird. Alle hierin beschriebenen detaillierten Erfindungsbeschreibungen und vorzugsweisen Ausführungsformen beziehen sich auf alle Aspekte der Erfindung.

### Detaillierte Beschreibung der Erfindung

Die Erfindung betrifft eine schonende Rotor-Stator Behandlung von Pflanzen oder Pflanzenzellen, die entgegen der ansonsten üblichen Homogenisierung Material aus dem Apoplasten der Zellen bzw. Pflanzen ablöst. Die Protoplasten sollen hierbei weitgehend intakt bleiben um Kontaminationen des zu isolierenden exprimierten Materials durch Zellbestandteile aus dem Zellinneren der Protoplasten zu vermeiden. Daher wird erfindungsgemäß die Intensität und Dauer der Behandlung mit dem Rotor-Stator limitiert. Es hat sich herausgestellt, dass durch eine Rotor-Stator Behandlung mit geringen Energieeinträgen - bestimmt als spezifische Wärme oder Wärmeleistung - ein zufriedenstellendes Ablösen des gewünschten exprimierten Materials möglich ist. Ausgezeichnete Resultate konnten bei einer durch Rotation des Rotors eingebrachte spezifische Wärme des Rotor-Stators von maximal 3 kJ pro kg des flüssigen Mediums und pro g/l Trockenmasse der Pflanzenzellen sowie bei einer durch Rotation des Rotors eingebrachten spezifischen Wärmeleistung in das Medium von maximal 1,5 kJ pro kg des flüssigen Mediums pro Minute und pro g/l Trockenmasse der Pflanzenzellen festgestellt werden. Für den Erfindungsgedanken gleichwertig, wurden ebenso gute Resultate ermittelt bei durch Rotation des Rotors eingebrachter Wärme des Rotor-Stators von maximal 30 kJ pro kg des flüssigen Mediums sowie bei einer eingebrachten Wärmeleistung in das Medium von maximal 1,5 kJ pro kg des flüssigen Mediums und pro Minute. Erfindungsgemäß werden dabei die Pflanzen oder Pflanzenzellen nicht homogenisiert, sondern nur soweit behandelt, sodass das exprimierte Material von der Oberfläche bzw. aus dem Apoplasten abgelöst wird.

Mit dem erfindungsgemäßen Verfahren wird exprimiertes Material von der Oberfläche der Zellen bzw. dem Apoplasten (Gesamtheit der Zellwände und der Interzellularraum) gewonnen. Derartiges Material gelangt an diese Stellen üblicherweise über einen sekretorischen Weg und ist zumeist auch im Kulturmedium der Pflanzenzellen zu finden. Allerdings wurde im Laufe der Erfindung festgestellt, dass große Mengen des sekretierten Materials an der Oberfläche, insb. an der Zellwand bzw. dem Apoplasten anhaftet. Dieses anhaftende Material wird erfindungsgemäß gewonnen, wobei durch das erfindungsgemäße Verfahren Produktionssteigerungen erzielt werden konnten. Ausbeutesteigerung im Vergleich zu Verfahren ohne Rotor-Stator Behandlung, i.e. Isolierung des sekretierten exprimierten Materials, um das 10-fache konnte beobachtet werden.

Die Behandlungsintensität, -dauer und Kapazität des Rotor-Stators werden innerhalb der erfindungsgemäßen maximalen Parameter gewählt, um eine ausreichende Gewinnung des exprimierten Materials (gewünschtes Produkt) zu erhalten. Allerdings wird Behandlungsintensität, -dauer und Kapazität, welche die eingebrachte Energie darstellt, quantifiziert als Wärme oder spezifische Wärme, limitiert, da bei höheren Energieeinträgen Produktkontaminationen auftreten.

Vorzugsweise ist die durch Rotation des Rotors eingebrachte spezifische Wärme des Rotor-Stators maximal 3 kJ pro kg des flüssigen Mediums und pro g/l Trockenmasse der Pflanzenzellen (abgekürzt 3 kJ/kg/(g/l) oder 3 kJ/kg/g/l). Insbesondere bevorzugt kann die spezifische Wärme geringer gehalten werden, um allfällige Restkontamination weiter zu reduzieren. So ist vorzugsweise die eingebrachte spezifische Wärme des Rotor-Stators maximal 2,75 kJ/kg/(g/l), oder weiter bevorzugt maximal 2,5 kJ/kg/(g/l), maximal 2,25 kJ/kg/(g/l), maximal 2 kJ/kg/(g/l), maximal 1,75 kJ/kg/(g/l), maximal 1,5 kJ/kg/(g/l), maximal 1,25 kJ/kg/(g/l), oder maximal 1 kJ/kg/(g/l).

Unabhängig von einem Bezug auf die Pflanzenmenge wurde erfindungsgemäß auch die optionale Kenngröße der eingebrachten Wärme festgestellt. Diese ist in manchen Ausführungsformen relevant, da der Rotor-Stator einen Energieeintrag in das Zellmedium unabhängig von den Pflanzenzellen liefert, welcher sich als Erwärmung äußern kann. Vorzugsweise ist die durch Rotation des Rotors eingebrachte Wärme des Rotor-Stators maximal 30 kJ pro kg des flüssigen Mediums (abgekürzt kJ/kg), vorzugsweise maximal 25 kJ/kg, maximal 20 kJ/kg, maximal 15 kJ/kg oder maximal 10 kJ/kg.

Diese eingebrachte spezifische Wärme oder eingebrachte Wärme kann z.B. durch zeitlich limitierte Behandlungsdauern und/oder der Intensität der Behandlung, eingestellt werden (ebenso die Parameter der spezifischen Wärmeleistung oder der Wärmeleistung):
Im erfindungsgemäßen Verfahren wird der Rotor-Stator bei einer geringen Intensität betrieben, z.B. bei geringer Drehzahl. Die bei einer bestimmten Intensität eingebrachte Wärme (bzw. Wärmeleistung) eines bestimmten Verfahrens bei gewählten Parametern kann in einem Vergleichsversuch z.B. durch Temperaturerhöhung von Wasser oder eines anderen Mediums mit bekannter Wärmekapazität gemessen werden. Bei einer Bestimmung der Verfahrenswärme sollten andere Temperatur-beeinflussenden Effekte, insb. von Temperaturverluste ausgeschlossen oder rechnerisch berücksichtig werden können, um so zur Wärme oder Wärmeleistung des Rotor-Stators selbst zu gelangen; bevorzugt wird die Wärme oder Wärmeleistung in einem Dewar-Gefäß bestimmt.

Unabhängig vom Gerät wurde die eingebrachte spezifische Wärmeleistung oder die eingebrachte Wärmeleistung als relevant erkannt ("spezifisch" bezieht sich wie oben auf den optionalen Bezug auf die Pflanzenmaterialmenge). Vorzugsweise ist die eingebrachte spezifische Wärmeleistung in das Medium maximal 1,5 kJ pro kg des flüssigen Mediums pro Minute und pro g/l Trockenmasse der Pflanzenzellen (abgekürzt kJ/kg/min/(g/l) oder kJ/kg/min/g/l). Insbesondere bevorzugt ist diese spezifische Wärmeleistung maximal 1,25 kJ/kg/min/(g/l), maximal 1 kJ/kg/min/(g/l), maximal 0,8 kJ/kg/min/(g/l), maximal 0,6 kJ/kg/min/(g/l), maximal 0,5 kJ/kg/min/(g/l), maximal 0,4 kJ/kg/min/(g/l), maximal 0,3 kJ/kg/min/(g/l), maximal 0,2 kJ/kg/min/(g/l), maximal 0,15 kJ/kg/min/(g/l), maximal 0,125 kJ/kg/min/(g/l), oder maximal 0,1 kJ/kg/min/(g/l). Analog dazu ist bevorzugt die eingebrachte Wärmeleistung in das Medium maximal 1,5 kJ pro kg des flüssigen Mediums und pro Minute (abgekürzt kJ/kg/min). Vorzugsweise ist diese Wärmeleistung maximal 1,25 kJ/kg/min, maximal 1 kJ/kg/min, maximal 0,8 kJ/kg/min, maximal 0,6 kJ/kg/min, maximal 0,5 kJ/kg/min, oder maximal 0,4 kJ/kg/min.

Je intensiver oder andauernder die Behandlung, desto größer ist auch die gewonnene Materialmenge. Vorzugsweise ist die durch Rotation des Rotors eingebrachte Wärme (des Rotor-Stators) mindestens 1 kJ pro kg des flüssigen Mediums, insbesondere bevorzugt mindestens 2 kJ/kg, und/oder die spezifische Wärme des Rotor-Stators mindestens 0,1 kJ pro kg des flüssigen Mediums und pro g/l Trockenmasse der Pflanzenzellen, insbesondere bevorzugt mindestens 0,2 kJ/kg/(g/l).

Vorzugsweise ist die durch Rotation des Rotors eingebrachte Wärmeleistung in das Medium mindestens 0,2 kJ pro kg des flüssigen Mediums und pro Minute, vorzugsweise mindestens 0,4 kJ/kg/min, und/oder die spezifische Wärmeleistung in das Medium mindestens 0,02 kJ pro kg des flüssigen Mediums pro Minute und pro g/l Trockenmasse der Pflanzenzellen, vorzugsweise mindestens 0,04 kJ/kg/min/ (g/l) .

Das exprimierte Material enthält vorzugsweise Proteine. Proteine, insbesondere rekombinant exprimierte Proteine, können gezielt mit entsprechenden Signalsequenzen zum sekretorischen Weg gesteuert werden und somit zur Anreicherung an der Oberfläche oder dem Apoplasten gelenkt werden. Vorzugsweise ist das exprimierte Material im Apoplast der Pflanzenzellen, aus dem es mit dem erfindungsgemäßen Verfahren gewonnen werden kann. Ebenso bevorzugt ist das exprimierte Material sekretiertes Material, vorzugsweise durch die Zellmembran oder Zellwand sekretierte Proteine.

Erfindungsgemäß wird ein Rotor-Stator verwendet, um die Pflanzenzellen im flüssigen Medium zu bearbeiten um das exprimierte Material zu gewinnen.

Ein Rotor-Stator umfasst mindestens einen Rotor, welcher durch seine Drehbewegung Scherkräfte auf die Pflanzenzellen ausübt. Durch die Scherkräfte werden die Oberfläche der Pflanzenzellen bzw. der Apoplast und die Zellwände (strukturell) gelockert, mechanisch beeinflusst bzw. abgerieben oder teilweise oberflächlich abgetragen.

Der Rotor rotiert im Vergleich zu einem Stator. Der Stator kann eine Gehäuseummantelung oder ein Gegenstück zum Rotor sein. Der Rotor kann Schneid- oder Scherelemente, mit Kanten oder Scherflächen, aufweisen. Eine übliche Konstruktion weist eine Kammstruktur auf, wobei eine Vielzahl Schervorsprüngen (z.B. Zähne oder Zinken), welche meist parallel zur Rotationsachse angeordnet sind, die Scher- oder Schneidwirkung ausüben. Eine "Vielzahl" können hier beispielsweise 2, 3, 4, 5, 6, 7, 8 oder mehr Schervorsprünge sein.

Der Stator kann als Gegenstück zum Rotor und insbesondere seinen Schneid- oder Scherelementen angeordnet sein. Optional kann der Stator ähnlich dem Rotor eigene Schneid- oder Scherelemente aufweisen, z.B. ebenso in Kammstruktur. Derartige Konstruktionen sind in Stabhomogenisatoren, wie in DE 10 2005 031 459 A1 beschrieben, bekannt.

In anderen Ausführungsformen kann der Stator eine Gehäusestruktur sein, wie z.B. für Durchflusshomogenisatoren üblich. Ein Beispiel eines Durchfluss Rotor-Stators ist in WO 2009/062610 A1 beschrieben.

Beispiele für Rotor-Statoren sind ein Stabhomogenisierer oder eine Scherpumpe. Scherpumpen werden insbesondere für Durchflusssysteme eingesetzt.

Vorzugsweise ist zwischen Rotor und Stator ein Spalt, z.B. mindestens in Größe einer Pflanzenzelle oder mehr, sodass Pflanzenzellen zumindest in Form des Protoplasten zwischen Rotor und Stator passieren können. Geeignete Spaltgrößen sind 50 µm, 70 µm, 80 µm, 100 µm, 150 µm oder mehr sowie jeder Bereich zwischen diesen Distanzen. Vorzugsweise bis zu maximal 500 µm oder bis zu 300 µm oder bis zu 200 µm.

Wie angemerkt, wird die Intensität gering gehalten, um die Pflanzenzellen in Form des Protoplasten zu schonen, um Kontaminationen des sekretierten exprimierten Materials, das geerntet werde soll, durch Zellinneres zu vermeiden oder zu reduzieren. Bei üblichen Rotor-Stator Modellen wird dazu die Drehzahl reduziert, z.B. in Ausführungsformen, wobei der Rotor mit einer Drehzahl von maximal 15000 Umdrehungen pro Minute betrieben wird, vorzugsweise 1000 bis 15000 Umdrehungen pro Minute. Mögliche Drehzahlen sind 3000 bis 14000, 4000 bis 13000, 5000 bis 12000 oder 6000 bis 11000, Umdrehungen pro Minute.

Die Pflanzenzellen können in einem Behälter vorliegen, in dem der Rotor-Stator eingebracht wird. Hierzu kann der Rotor-Stator in einen Behälter mit dem Medium eingebracht werden. Diese "batch"-Konstruktion (für ein nicht-kontinuierliches Verfahren) wird insbesondere bei Stabhomogenisierern verwendet. Für größere Maßstäbe werden vorzugsweise Durchfluss-Rotor-Statoren verwendet. Gemäß dieser Ausführungsform kann der Rotor-Stator einen Innenraum aufweisen, welcher mindestens je einen Ein- und Ablauf hat, durch die kontinuierlich das flüssige Medium durch den Innenraum befördert wird. Ein Beispiel hierfür ist die Scherpumpe für ein kontinuierliches Verfahren.

Vorzugsweise begrenzt der Stator ein Volumen von 10 cm³ (0,01 1) bis 1 m³ (1000 1). Bevorzugte Volumina sind 0,1 1 bis 800 l, oder 0,5 1 bis 600 1 oder 1 1 bis 400 l, 2 1 bis 200 l. Bevorzugt sind Volumina bis 100 l, insbesondre bevorzugt 0,65 1 bis 50 l, z.B. 1 1 bis 40 l. Diese Volumina sind insbesondere zur Behandlung von Kulturmedien pflanzlicher Zellen bestens geeignet.

Vorzugsweise ist die Menge des behandelten flüssigen Mediums bis zu 50000 kg, vorzugsweise 0,5 g bis 50000 kg, z.B. 1g bis 25000, 2 g bis 10000 kg, 5 g bis 5000 kg, 10 g bis 2500 kg, 20 g bis 1000 kg, 30 g bis 500 kg, 50 g bis 250 kg, 100 g bis 100 kg, 200 g bis 50 kg, 500 g bis 250 kg, 1 kg bis 100 kg, 2 kg bis 50 kg oder 4 kg bis 20 kg. Derartige Mengen werden vorzugsweise im nicht-kontinuierlichen Verfahren pro Durchgang verwendet.

Vorzugsweise liegen die pflanzlichen Zellen in einer Konzentration von 0,2 g/l bis 60 g/l im flüssigen Medium vor (Masse der Pflanzenzellen als Trockenmasse). Darin bevorzugte Konzentrationen der pflanzlichen Zellen (stets Trockenmasse) sind 0,5 g/l bis 50 g/l, 1 g/l bis 40 g/l, 2 g/l bis 30 g/l, 4 g/l bis 20 g/l, insbesondere bevorzugt etwa 10 g/l, z.B. 5 g/l bis 15 g/l. Diese Pflanzenzellkonzentrationen sind besonders effizient mit dem Rotor-Stator bearbeitbar.

Vorzugsweise werden die Pflanzenzellen 2 min bis 150 min mit dem Rotor-Stator behandelt. Im kontinuierlichen Verfahren beziehen sich diese Zeiten auf die durchschnittliche Behandlungszeit der Pflanzenzellen. Bevorzugte Zeiten sind insbesondere 3 min bis 120 min, 5 min bis 100 min, 8 min bis 80 min, 10 min bis 60 min, oder besonders bevorzugt 12 min bis 40 min. Bei großen Kulturvolumen können auch längere Rotor-Stator Behandlungen vorgenommen werden. Vorzugsweise sind weitere mögliche Zeiten 1 h bis 24 h, vorzugsweise 2 h bis 20 h, 3 h bis 16 h, 4 h bis 12 h. Somit betreffen alle vorzugsweisen Behandlungszeiten den Bereich 3 min bis 24 h, und jeden Bereich zwischen den genannten Behandlungszeiten oder auch länger.

Vorzugsweise sind die Pflanzenzellen in einer Suspensionskultur kultivierbar. Diese Suspension kann direkt als flüssiges Medium im erfindungsgemäßen Verfahren bearbeitet werden. Alternativ kann Moos auch zuerst isoliert, z.B. aus einer festen Kultur, Flüssigkultur oder Suspensionskultur, und dann in einem wässrigen Medium mit geeigneten Bedingungen für den Einsatz der Rotor-Stator Behandlung suspendiert werden. Pflanzen, die für das erfindungsgemäße Verfahren besonders geeignet sind, sind nicht-verholzende Pflanzen. Vorzugsweise Pflanzen sind Algen und Moose, insbesondere Bryophyten. Vorzugsweise ist die Bryophytenpflanze oder -zelle ein Moos, vorzugsweise *P. patens.* Der Bryophyt kann jeder beliebige Bryophyt sein, wird aber vorzugsweise aus Moos, Lebermoos oder Hornmoose ausgewählt, insbesondere bevorzugt aus der Klasse *Bryopsida* oder den Gattungen *Physcomitrella, Funaria, Sphagnum, Ceratodon, Marchantia* und *Sphaerocarpos.* Speziell bevorzugt ist *Physcomitrella patens.* Am meisten bevorzugt wird das erfindungsgemäße Verfahren unter Verwendung von Zellen aus Pflanzengewebe wie Protonema des Laubmooses *Physcomitrella patens* durchgeführt. Bevorzugte Algen sind ausgewählt aus den Grünalgen, z.B. aus der Ordnung der Chlorellales, bevorzugt der Familie der Chlorellaceae, mehr bevorzugt dem Genus *Auxenochlorella* oder *Chlorella,* insbesondere *Chlorella vulgaris,* und der Ordnung der Volvocales, bevorzugt der Familie der Haematococcaceae, mehr bevorzugt dem Genus *Haematococcus,* insbesondere *Haematococcus pluvialis* und der Ordnung der Eustigmatales, bevorzugt der Familien der Loboceae, Chlorobothryaceae, Pseudocharaciopsidaceae und Eustigmataceae. Weitere bevorzugte Pflanzen sind Tabak, Bohnen oder Linsen. Bevorzugt ist die Pflanze eine Wasserpflanze, z.B. der Gattungen *Lemna, Spirodela, Landoltia, Wolffia* oder *Wolffiella.*

Gegenstand der Erfindung sind Pflanzenzellen. "Pflanzenzelle", wie sie hierin verwendet wird, kann sich auf eine isolierte Zelle, eine singularisierte Zelle, aber auch auf eine Zelle in oder aus einem pflanzlichen Gewebe beziehen, vorzugsweise ein Gewebe, ausgewählt aus Kallus, Protonema, Phloem, Xylem, Mesophyll, Stamm, Blättern, Thallus, Chloronema, Rhizoid oder Gametophor, oder eine Zelle in einem Pflanzenorganismus.

Im erfindungsgemäßen Verfahren hat das Medium vorzugsweise einen physiologischen pH-Wert, insbesondere um wie bereits beschrieben, die Protoplasten (Zellbestandteile innerhalb der Zellwand, insb. ab der Zellmembran) zu schonen, um Kontaminationen des exprimierten Materials im Apoplasten/an der Oberfläche der Zellen zu vermeiden. Der pH-Wert des flüssigen Mediums ist vorzugsweise zwischen 3,5 und 8,5, insbesondere bevorzugt zwischen 4 und 8, oder zwischen 4,5 und 7, oder zwischen 5 und 6,5, insbesondere zwischen 5,5 und 6, oder Kombinationen dieser Bereiche, wie ein pH-Wert von 5 bis 8.

Ebenfalls um die Protoplasten zu schonen ist vorzugsweise die Osmolarität des flüssigen Mediums physiologisch, insbesondere um einen Quellstress zu vermeiden, z.B. bei zu niederer Osmolarität. Ggf. kann auch ein oberes Limit der Osmolarität vorgesehen werden, um einen osmotischen Schrumpfstress zu vermeiden. Vorzugsweise hat das Medium eine Osmolarität von mindestens 0,1 osmol/L, oder vorzugsweise mindestens 0,150 osmol/L. Die Osmolarität kann durch gelöste Stoffe, wie Salze oder andere Mediumkomponenten, wie Zucker oder Zuckeralkohole, eingestellt werden. Vorzugsweise wird ein Alkalimetallsalz wie Na⁺ und/oder K⁺, vorgesehen. Vorzugsweise wird als Anion ein Halid, wie Cl⁻ oder F⁻ oder I⁻, ein Phosphat oder ein Acetat vorgesehen. Weiter möglich sind Pufferkomponeten, wie Tris (Tris(hydroxymethyl)-aminomethan).

Weiters können zur weiteren Schonung der Protoplasten oberflächenaktive Polymere zum flüssigen Medium zur Rotor-Stator Behandlung vorgesehen werden. Oberflächenaktive Polymere sind beispielsweise in WO 2013/156504 A1 beschrieben, und umfassen vorzugsweise ungeladene Polymere, wie Emulgatoren, z.B. Polyalkylglykol, insb. Polyethylenglykol. Das Polymer ist insbesondere ein nichtionisches wasserlösliches oberflächenaktives Polymer. Vorzugsweise ist es nicht denaturierend auf Proteine. Beispiele sind Polymere oder Copolymere ausgewählt aus Polyethern wie Polyalkylglykole, Polysorbate oder Polyvinylpyrrolidon, Polyvinylalkohol, wasserlösliche Cellulosederivate wie Hydroxypropylcellulose, Hydroxypropylmethylcellulose, Carboxymethylcellulose oder Hydroxyethylcellulose, VinylpyrrolidonVinylacetat-Copolymer (Copovidone), Polyvinylacetat, partiell hydrolysierter Polyvinylalkohol, Polyvinylalkohol-Polyethylenglykol-Copolymere und Mischungen daraus. Weitere Möglichkeiten sind Polysorbat, z.B. Polyoxyethylen-sorbitan-monolaurat, Polyoxyethylen-sorbitan-monooleat, Polyoxyethylen-sorbitan-monopalmitat,

Polyoxyethylen-sorbitan-monostearat, Polyoxyethylen-sorbitan-tristearat, vorzugsweise Polysorbat 80 (Polyoxyethylen(20)-sorbitan-monooleat, Tween^{®} 80), Polyoxyethylen(40)stearat. Das oberflächenaktive Polymer liegt vorzugsweise in einer Konzentration von mindestens 0,05 gew.-%, speziell bevorzugt von mindestens 0,08 %, mindestens 0,1 % oder mindestens 1,5 %, im Medium vor (alle %-Angaben in gew.-%). Das Molgewicht des oberflächenaktiven Polymers, z.B. PEG und dergleichen, ist vorzugsweise mindestens 500 Da, insbesondere bevorzugt mindestens 1.000 Da, mindestens 1.500 Da, mindestens 2.000 Da, mindestens 3.000 Da, mindestens 4.000 Da, mindestens 6.000 Da, mindestens 8.000 Da, mindestens 10.000 Da, mindestens 20.000 Da, mindestens 30.000 Da. Speziell bevorzugt ist das Molgewicht zwischen 500 Da und 2.000.000 Da, bevorzugt zwischen 1.000 Da und 200.000 Da oder zwischen 1.200 Da und 80.000 Da.

Das flüssige Medium ist vorzugsweise wässrig, insbesondere Wasser oder für Zell-verträgliche Wassermischungen. Insbesondere kann es ein Kulturmedium für (und mit) Pflanzenzellen sein, sofern die Pflanzen zuvor nicht davon getrennt werden.

Vorzugsweise wird das exprimierte Material vor der Behandlung der Pflanzenzellen mit dem Rotor-Stator exprimiert, sodass es sich an der Oberfläche oder im Apoplast der Pflanzenzellen ansammelt. Dabei können die Pflanzenzellen kultiviert und/oder wachsen gelassen werden, z.B. in einem Medium unter Wachstumsbedingungen für Pflanzen (Nährmedien, Licht), wie allgemein bekannt (s. z.B. Frank et al. Plant Biol 7, (2005):220-227). Das Exprimieren bzw. Kultivieren wird vorzugsweise für 13 min bis 1 Monat (30 Tage) oder länger, wie 2 Monate (60 Tage), vorgenommen, z.B. 1h bis 22 Tage, oder 5 h bis 15 Tage, z.B. 10h bis 7 Tage oder 20h bis 3 Tage. In einer kontinuierlichen Zellkultur mit regelmäßiger Zellentnahme zur Produktgewinnung (Exprimiertes Material), können diese Zeitbereiche oder Mindestzeiten einer Durchschnittlichen Zeit einer Zelle in der Kultur entsprechen.

Weitere Verfahren, die den Protoplasten beschädigen oder lysieren oder homogenisieren sollen vermieden werden. Die Zellwand wird vorzugswese nicht lysiert, insbesondere nicht enzymatisch und/oder nicht chemisch und/oder nicht osmolytisch und/oder nicht durch Ultraschall lysiert. Die Zellwand soll vorzugsweise - abgesehen von der erfindungsgemäßen Rotor-Stator Behandlung - unangetastet oder intakt bleiben. Insbesondere soll die Zellmembran (Protoplast) intakt bleiben, wobei beim erfindungsgemäßen Verfahren die Vitalität der Zellen keine besondere Rolle spielt, jedoch sollen Kontaminationen des flüssigen Mediums durch Komponenten des Zellinnenraums, insbesondere des Zellplasmas, vermieden werden.

Die vorliegende Erfindung wird weiters durch die folgenden Figuren und Beispiele beschrieben, ohne auf diese Ausführungsformen der Erfindung beschränkt zu sein.

### Figuren:

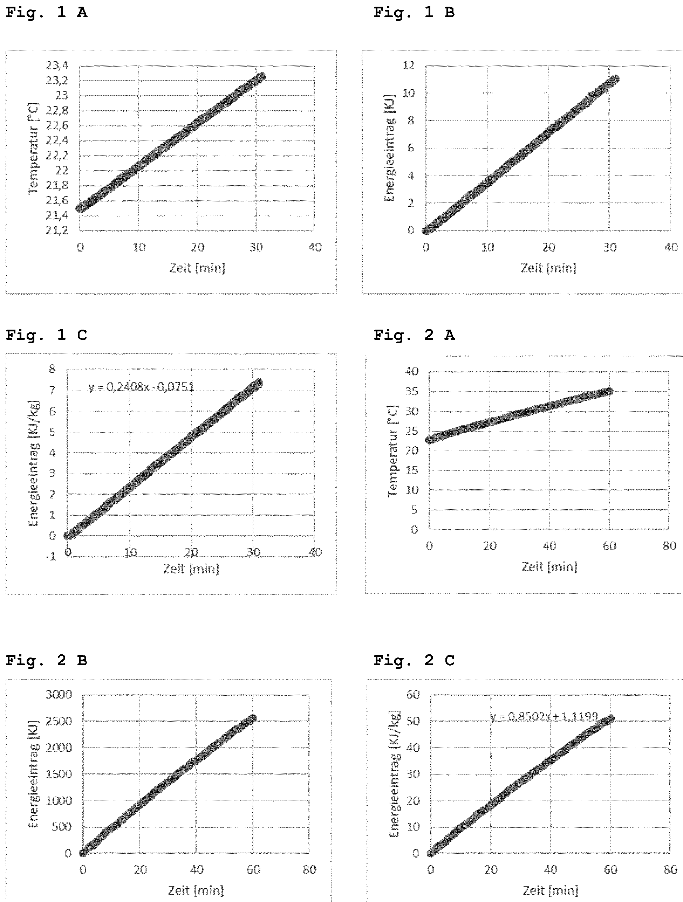

**Fig. 1****:** Bestimmung des Energieeintrags in Wasser durch Homogenisierer Ultraturraxstab T25 (IKA/Staufen). (A) Temperaturverlauf in 1,5 1 Wasser bei einer Drehzahl von 10000 rpm. (B) Berechneter Energieeintrag [KJ]. (C) Berechneter Energieeintrag [KJ/kg].
**Fig. 2****:** Bestimmung des Energieeintrags in Wasser durch Homogenisierer Shearpump FSP712VC-2.2kW-FU (Fristram/Hamburg). (A) Temperaturverlauf in 50 1 Wasser bei einer Drehzahl von 2800 rpm. (B) Berechneter Energieeintrag [KJ]. (C) Berechneter Energieeintrag [KJ/kg].

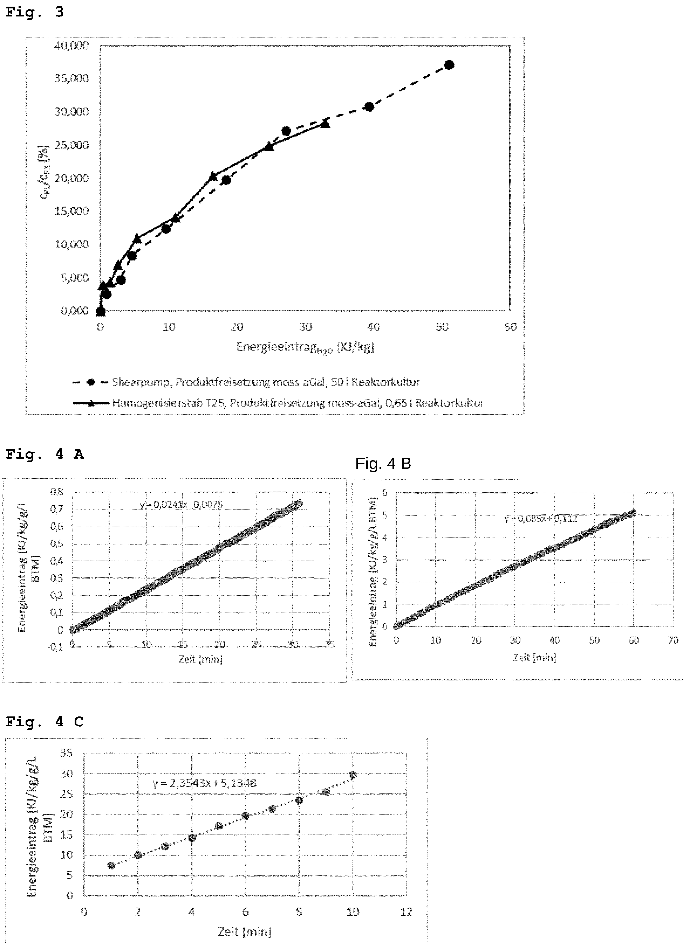

**Fig. 3****:** Prozentuale Freisetzung von Biomasse-gebundenem Produkt (moss-aGal) während der Behandlung mit Shearpump (unterbrochene Linie) und Ultraturraxstab T25 (durchgezogene Linie). Die Shearpumpenbehandlung der Reaktorkultur erfolgte in einem Volumen von 50 l. Die Behandlung der Reaktorkultur mit Turraxstab T25 erfolgte in einem Volumen von 0,65 l.
**Fig. 4****:** Spezifischer Energieeintrag durch Ultraturraxstab T25 (A) und Shearpump (B), beide für 10 g/l Biotrockenmasse. Spezifischer Energieeintrag gemäß Vergleichsbeispiel Ultraturraxstab T25 bei 19000 rpm mit 1 g/l Biotrockenmasse (C)

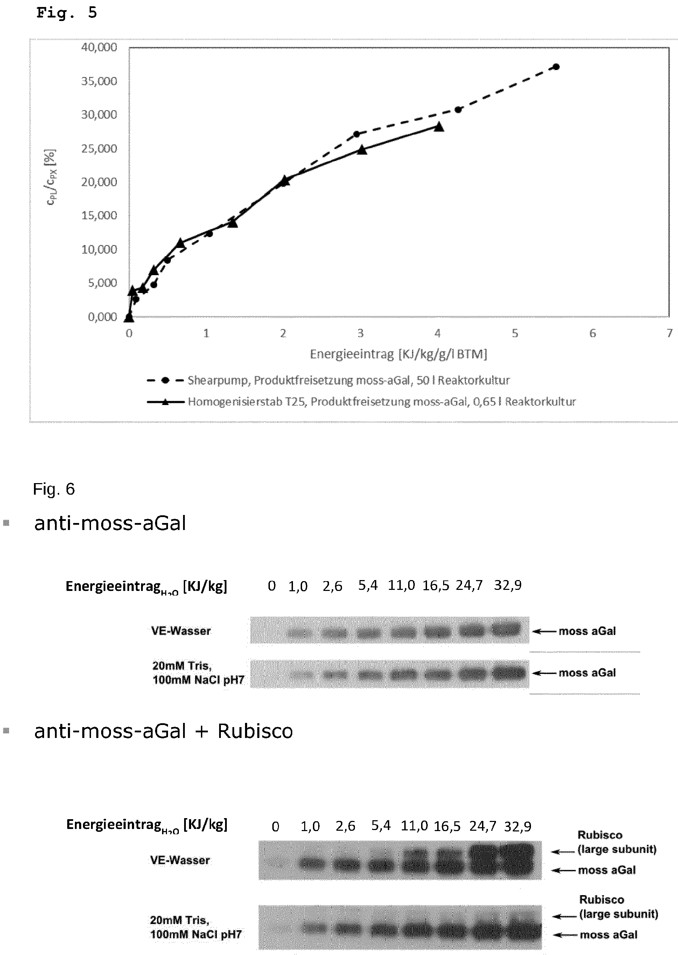

**Fig. 5****:** Prozentuale Freisetzung von Biomasse-gebundenem Produkt (moss-aGal) während der Behandlung mit Shearpump (unterbrochene Linie) und Ultraturraxstab T25 (durchgezogene Linie). Die Shearpumpenbehandlung der Reaktorkultur erfolgte in einem Volumen von 50 1. Die Behandlung der Reaktorkultur mit Turraxstab T25 erfolgte in einem Volumen von 0,65 l. Biomassen: 9,2 g/L (Shearpump), 8,2 g/L (T25)
**Fig. 6****:** Western Blot Analyse zur Produktfreisetzung (moss-aGal) im Vergleich zur Freisetzung von intrazellulären Markerproteinen (Rubisco, large subunit). Intrazelluläre Proteine in salzhaltigen Medien (z.B. 20 mMTris, 100 mM NaCl, pH=7) bei Energieeinträgen bis 32,9 KJ/kg in minimalen Mengen detektierbar. Unter osmotischen Stressbedingungen (demineralisiertes Wasser) ab ca. 10 KJ/kg detektierbar. Zielproteinmengen (moss-aGal) steigen in salzhaltigen Medien und unter osmotischen Stressbedingungen entsprechend der eingetragenen Energie an.

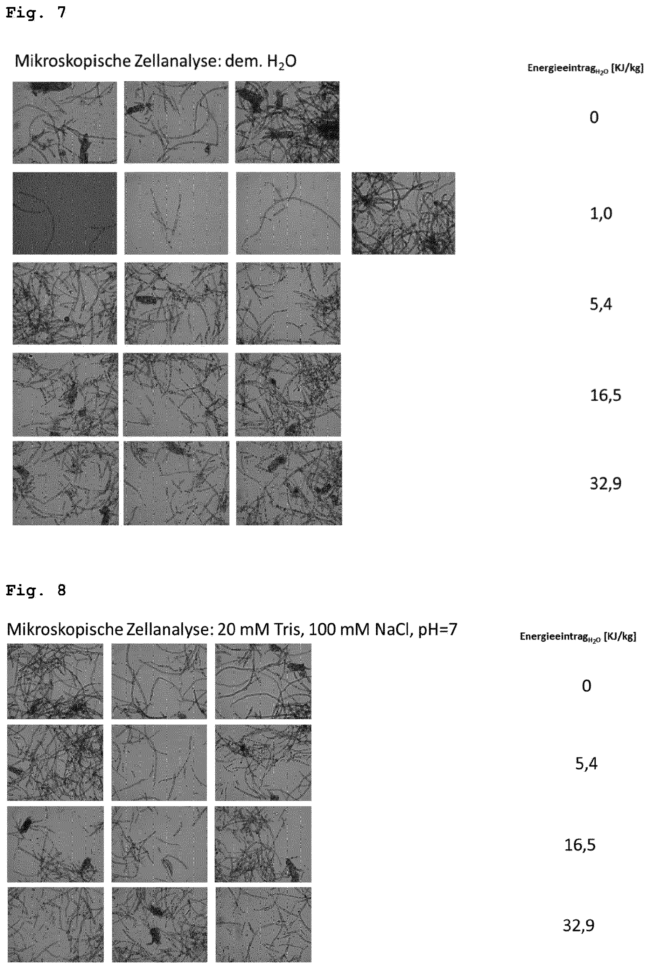

**Fig. 7****:** Mikroskopische Analyse des T25 Prozesses in demineralisiertem H2O. Bis zu einem Energieeintrag von 16,5 kJ/kg behalten die Mooszellen die Integrität. Bei dem Energieeintrag von 32,9 KJ/kg ist die Integrität der Zellen vorhanden, jedoch sind offensichtlich mehr Partikel vorhanden, die auf einen beginnenden Zellaufschluss hinweisen.
**Fig. 8****:** Mikroskopische Analyse des T25 Prozesses in salzhaltigem Puffer (20mM Tris, 100 mM NaCl, pH=7). Bis zu einem Energieeintrag von 16,5 kJ/kg behalten die Mooszellen die Integrität. Bei dem Energieeintrag von 32,9 KJ/kg ist die Integrität der Zellen vorhanden, jedoch sind offensichtlich mehr Partikel vorhanden, die auf einen beginnenden Zellaufschluss hinweisen.

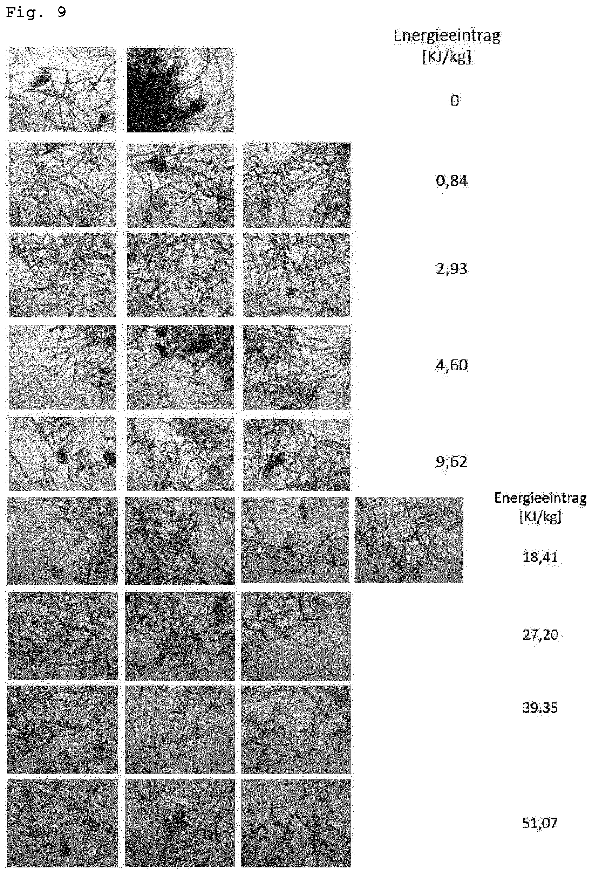

**Fig. 9****:** Mikroskopische Analyse des Shearpump Prozesses in salzhaltigem Medium (Reaktorkultur). Bis zu einem Energieeintrag von 18,41 kJ/kg behalten die Mooszellen die Integrität. Ab einem Energieeintrag von 27,20 KJ/kg ist die Integrität der sichtbaren Zellen vorhanden, jedoch sind offensichtlich mehr Partikel vorhanden, die auf einen beginnenden Zellaufschluss hinweisen.

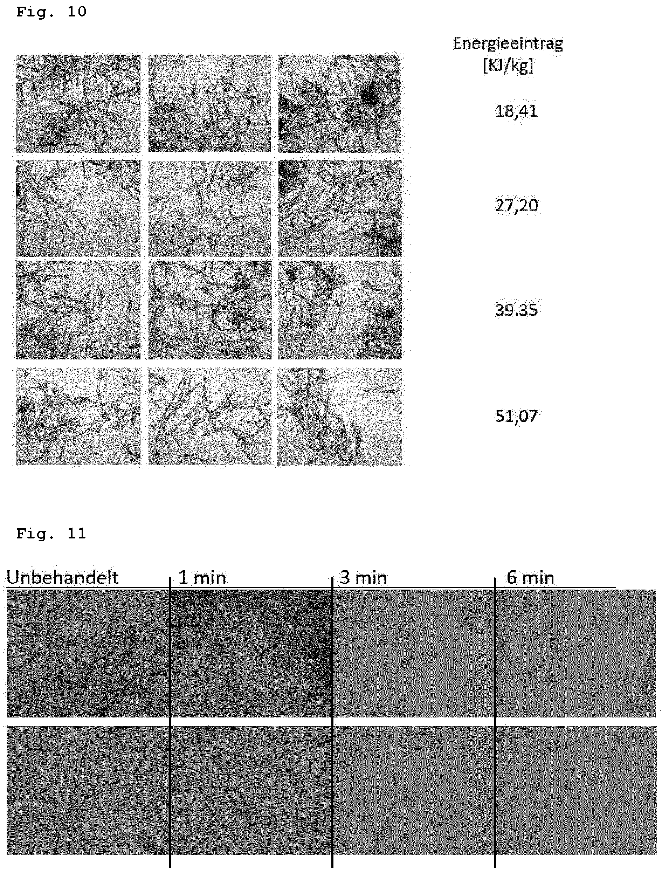

**Fig. 10****:** Mikroskopische Analyse des Shearpump Prozesses in demineralisiertem H2O. Bis zu einem Energieeintrag von 9,62 kJ/kg behalten die Mooszellen die Integrität. Ab einem Energieeintrag von 18,41 KJ/kg ist die Integrität der Zellen vorhanden, jedoch sind offensichtlich mehr Partikel vorhanden, die auf einen beginnenden Zellaufschluss hinweisen.
**Fig. 11****:** Mikroskopische Analyse von Mooszellen in einem Ultraschall Prozess als vergleichende Bilder eines Zellaufschlusses. Die Zellen verlieren bereits nach kurzer Zeit (1 min Ultraschallbehandlung) ihre Integrität. Bereits nach 3 min sind ausschließlich Zellfragmente und leere Zellhüllen zu sehen. (Leistung 100% in 50 ml Ansatz).

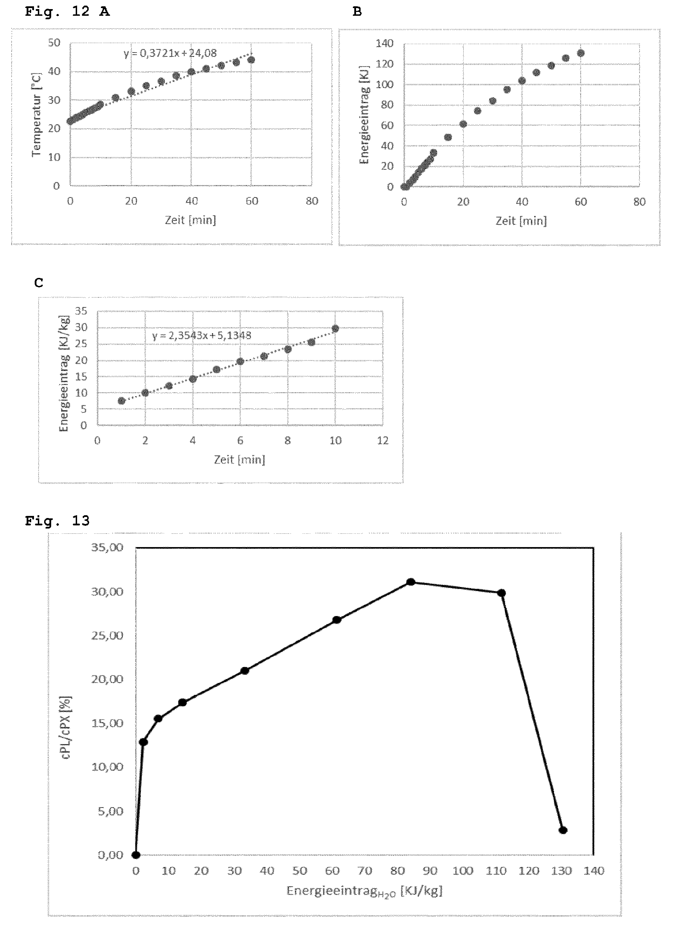

**Fig. 12****:** Vergleichsbeispiel: Bestimmung des Energieeintrags in Wasser durch Homogenisationswerkzeug T25 (IKA/Staufen) bei hoher Energie einer Drehzahl von 19000 rpm. (A) Temperaturverlauf in 1 l Wasser. (B) Berechneter Energieeintrag [KJ]. (C) Berechneter Energieeintrag [KJ/kg].
**Fig. 13****:** Vergleichsbeispiel: Deutlich schnellere Produktfreisetzung bei 19000 rpm. Insbesondere bis zu einem Energieeintrag von 7 KJ/kg. Ab einem Energieeintrag von 84 KJ/kg Produktverluste durch hohe Temperatur u. Scherstress.

### Beispiele:

### Beispiel 1: Bestimmung des Energieeintrags mittels Turraxstab und Shearpump

Als Maß für den Energieeintrag in wässrige Medien wurde die Temperatur als messbare Größe verwendet. Bei Turraxstab T25-S25N-18G (IKA Staufen) wurden 1,5 l H₂O in einem Dewar-Gefäß für 30 min bei 10.000 rpm dispergiert und der Temperaturverlauf vermessen. Die Raumtemperatur während der Versuchsdurchführung lag zwischen 20,5 und 20,7 °C. Über die spezifische Wärmekapazität (4190 J · kg⁻¹K⁻¹) von H₂O wurden Energieeintragsdaten berechnet. Energieeintragsdaten vergleichend zur Literatur (Orellana-Escobedo et al., Plant Cell Rep. 2015, 34(3), 425-433) wurden analog bei 19000 rpm durchgeführt.

Mit einer Scherpumpe (Shearpump FSP 712, Fristam Hamburg) wurden 50 l H₂O bei 2800 rpm aus einem Nalgene Vorlagegefäß über gewebestabilisierte PVC-Schläuche im Kreis gefördert. Die Temperaturmessung erfolgte im Nalgene Vorlagegefäß über einen Temperatursensor (Präzisionsthermometer, G002.1, Carl Roth). Der Versuchsaufbau wurde in einem auf 19 °C temperierten Raum realisiert. Temperaturverluste in die Umgebung wurden in diesem Versuchsansatz vernachlässigt. Über die spezifische Wärmekapazität (4190 J · kg⁻¹K⁻¹) von Wasser wurden Energieeintragsdaten berechnet.

### Beispiel 2: Herstellung einer Mooskultur

Moos Produktionsstämme wurden für 3-4 Wochen in 200 1 Single Use Bioreaktorbeuteln (Cellbag 200, GE Healthcare) auf Wave^{™} Rocking Motion Bioreaktoren (Wave200, Ge Healthcare) axenisch kultiviert. Als Kultivierungsparameter wurden Schüttelfrequenzen von 19 bis 25 rpm, Schüttelwinkel von 9 °, Temperaturen von 24-26 °C und eine Begasungsrate von 2 L/min und einer Anreicherung der Zuluft mit 2 % CO₂ verwendet. Zur Beleuchtung wurden 4 über dem Bioreaktorbeutel installierte LED-Module (Seriennummer 120268 bis 120282, Infors AG) mit LEDs der Lichtfarbe "warm white" verwendet. Die Moos Kultivierung erfolgte unter 24 h Dauerbeleuchtung. SM07 (100 mM NaCl, 6,6 mM KCL, 2,0 mM MgSO₄ x 7H₂O, 1,8 mM KH₂PO₄, 20,4 mM Ca(NO₃)₂ x 4H₂O, 0.05 mM Fe Na-EDTA, 4,9mM MES, 0,1 % (w/v) PEG4000, 100,26 µM H₃BO₃, 0,11 µM CoCl₂ x 6H₂O, 0,1 µM CuSO₄ x 5H₂O, 5 µM KI, 85,39 µM MnCl₂ x 4H₂O, 1,03 µM Na₂MoO₄ x 2H₂O, 0,11 mM NiCl₂ x 6H₂O, 0,04 Na₂SeO₃ x 5H₂O, 0,039 Zn-acetate x 2H₂O) supplementiert mit 1000x Nitsch Vitaminen (Nitsch vitamin mixture, Duchefa, entspr. Herstellerangaben) diente als Mineralsalzmedium. Der pH-Wert von 5-6 wurde über WAVEPOD I und Pump20 (GE Healthcare) über eine automatisierte Zugabe von 0,25 M H₂SO₄ und 0,25 M NaOH geregelt. Die Expression von rekombinanter α-Galactosidase (aGal oder α-Gal A) erfolgte wie in WO 2016/146760 A1 beschrieben ("moss-aGal").

### Beispiel 3: Freisetzung von Moos gebundenem Produkt und analytische Methoden

Zur Analyse des zeitlichen Verlaufs der Freisetzung von Moos gebundenem Produkt wurde die aus Beispiel 2 erhaltene Kultur mit Turraxstab T25-S25N-18G sowie mit Shearpump FSP 712 unterschiedlichen Energieeinträgen ausgesetzt. c_{PL}-Produktkonzentrations-bestimmungen (c_{PL}) für freigesetztes Produkt erfolgten mittels moss-aGal-ELISA (Biogenes/Deutschland). Eine Detektion des Zellaufschlussgrades erfolgte über mikroskopische Bildanalyse der Mooszellen (Mikroskop: Axiovert 200 oper Stemi SV11 mit Kamera AxioCam, Software AxioSoft und Kaltlichtquelle KL 1500 LCD (Carl Zeiss). Ein Vergleich zu mikroskopischen Bildern eines Totalaufschlusses war über mikroskopische Analysen eines 50 ml Ultraschallaufschlusses (Sonde: UW2070, Bandelin; Verstärker: HD 2070, Bandelin) bei 100 % Leistung über 20 min möglich. Auf molekularer Ebene wurde qualitativ über Western Blotting auf freigesetztes Produkt sowie intrazelluläres Markerprotein Rubisco anlaysiert. Als Primärantikörper wurden anti-aGal (H00002717-D01P, abnova) und anti-Rubisco (AS03037, Agrisera) verwendet sowie als Sekundärantikörper anti-Rabbit HRP (abcam, AS03037).

Für eine Analyse des Verhältnisses zwischen freigesetztem (c_{PL}) und freisetzbarem Moos gebundenem Produkt (c_{PX}) wurde die unbehandelte Kultur einem Zellaufschluss mittels Kugelmühle (Stahlkugeln: RB-3/G20W, Schleer; Kugelmühle: MM300, Retsch) unterzogen. Nach Abtrennung von Zellbruchstücken erfolgte die Produktkonzentrationsbestimmung mittels ELISA (Biogenes). Hierbei wurde aGal (α-Galactosidase, auch "moss-aGal"), ein in den apoplastischen Raum exprimiertes Protein, bestimmt.

### Beispiel 4: Ergebnisse und Diskussion

Die Energieeinträge (als Wärme in kJ/kg, kg bezogen auf das flüssige Medium) zweier unterschiedlicher Homogenisierer - ein Turraxstab T25-S25N-18G sowie mit Shearpump FSP 712 - in ein Kulturmedium (kg) wurden durch Temperaturmessungen festgestellt (Fig. 1-5). Hierbei wurden die Homogenisierer auf eine niedere Drehgeschwindigkeit eingestellt, sodass eine niedrige Wärmeleistung (in kJ/kg/min) erzielt wird. Die Gesamtwärme über einen bestimmten Zeitraum (0-60 min) konnte so festgestellt werden. Hierbei wurde der Energieeintrag in Wasser über den spezifischen Wärmekoeffizient von H₂O [4,182 KJ/kg*K] basierend auf der gemessenen Temperatur berechnet. Hierbei wurde die Wärme (Fig. 1-3) bzw. die spezifische Wärme bezogen auf die Trockenmasse der Pflanzenteile (Fig. 4-5) berechnet.

Fig. 3 und 5 zeigen die Produktfreisetung des gewünschten Proteins (rekombinante aGal aus Moos, "moss-aGal"), welche sich an der Oberfläche bzw. im Apoplasten anlagert. Mit zunehmender Behandlung steigt auch die Freisetzung.

In Vergleichsversuchen wurde der Turraxstab bei hoher Wärmeleistung betrieben, und zwar bei 19000 rpm (Fig. 4C, 12-13). Die Wärmeleistung war in etwa um einen Faktor 100 größer als bei der schonenden Behandlung bei 10000 rpm (vgl. Fig. 4 A und 4 C). Der Betrieb bei hoher Wärmeleistung führt rasch zur Produktfreisetzung allerdings auch zur Zerstörung der Zellen (Protoplasten), sodass extrazelluläre Produkte durch Bestandteile des Zellinnenraums kontaminiert werden. Diese Effekte treten bereits bei 1 Minute Behandlung auf.

Figur 6 zeigt die Qualität der Produkt Freisetzung des extrazellulären Proteins (moss aGal) und des intrazellulären Proteins Rubisco. Rubisco kommt in Pflanzenzellen in hohen Konzentrationen vor und wurde daher als hochsensibler Marker für den Austritt von Zellinhalten herangezogen. In den Experimenten in physiologischer Salzlösung zeigt sich bei höheren Energieeinträgen (Wärme) von über 32,9 kJ/kg eine zunehmende Freisetzung von Rubisco. In einem Vergleichsversuch mit demineralisiertem Wasser (VE Wasser) tritt die Verunreinigung mit Rubisco bereits früher auf, etwa ab 10 kJ/kg, da zusätzlich zur Scherbelastung durch den Homogenisierer osmolytische Effekte hinzukommen. Die unterschiedlichen Wärmen (Energieeinträge) wurden durch die Behandlungszeit gesteuert.

Die mikroskopische Analyse der Zellverbände nach Behandlung spiegelt diese Ergebnisse. Fig. 7 zeigt die Ergebnisse nach Behandlung mit dem Ultraturrax Rotor-Stator in demineralisiertem Wasser; Fig. 8 nach Behandlung mit dem Ultraturrax Rotor-Stator in physiologischen Bedingungen (20mM Tris, 100 mM NaCl, pH=7). Fig. 9 zeigt die Zellverbände nach Behandlung mit der Shearpump in physiologischen Bedingungen. Mit zunehmender Behandlungszeit (Wärme) nimmt die Zahl von Partikeln, vermutlich gebildet durch zerstörte Zellen, zu. Ab etwa 30 kJ/kg zeigt sich ein vermehrter Zellaufschluss. Zum Vergleich zeigt Fig. 10 die Versuche in demineralisiertem Wasser mit der Shearpump. Hier treten vergleichbare Partikel bereits bei etwa 20 kJ/kg auf.

Zum Vergleich zu den Fig. 7-10 zeigt Fig. 11 einen Zellaufschluss durch Ultraschall. Bereits nach 1 min verlieren die Zellen ihre Integrität.

Daraus ergibt sich, dass zum einen der Energieeintrag pro Zeiteinheit (Intensität der Rotation des Rotor-Stators; Wärmeleistung) limitiert werden soll, als auch der absolute Energieeintrag (Wärme) - hier im Experiment durch die Behandlungsdauer gesteuert. Diese Parameter könne als solche oder bezogen auf die Biomasse (Trockenbiomasse, TBM) herangezogen werden. Sinnvolle Kennzahlen eines schonenden Verfahrens, das möglichst viel der absorbierten bzw. Apoplastengebundenen Produkte ablöst und dabei die Protoplasten weitgehend intakt hält sind maximal 3 kJ/kg pro g/l Trockenmasse und maximal 1,5 kJ/kg/min pro g/l Trockenmasse oder maximal 30 kJ/kg und maximal 1,5 kJ/kg/min. Mögliche Behandlungszeiten bei derartigen niedrigen Wärmeleistungen sind 2 min bis 150 min - je nach Intensität der Rotation, in der Regel länger als die bisher üblichen kurzen aber intensiven Behandlungen.

## Patentansprüche

1. Ein Verfahren zum Ablösen von exprimiertem Material von der Oberfläche oder dem Apoplast von Pflanzenzellen, wobei die Pflanzenzellen in einem flüssigen Medium mit einem Rotor-Stator behandelt werden, wobei die durch Rotation des Rotors eingebrachte spezifische Wärme des Rotor-Stators maximal 3 kJ pro kg des flüssigen Mediums und pro g/l Trockenmasse der Pflanzenzellen und die eingebrachte spezifische Wärmeleistung in das Medium maximal 1,5 kJ pro kg des flüssigen Mediums pro Minute und pro g/l Trockenmasse der Pflanzenzellen beträgt.

2. Das Verfahren nach Anspruch 1, **dadurch gekennzeichnet, dass** das exprimierte Material im Apoplast der Pflanzenzellen ist.

3. Das Verfahren nach Anspruch 1 oder 2, **dadurch gekennzeichnet, dass** die durch Rotation des Rotors eingebrachte Wärme des Rotor-Stators mindestens 1 kJ pro kg des flüssigen Mediums beträgt, und/oder die spezifische Wärme des Rotor-Stators mindestens 0,1 kJ pro kg des flüssigen Mediums und pro g/l Trockenmasse der Pflanzenzellen beträgt.

4. Das Verfahren nach einem der Ansprüche 1 bis 3, **dadurch gekennzeichnet, dass** die durch Rotation des Rotors eingebrachte Wärmeleistung in das Medium mindestens 0,2 kJ pro kg des flüssigen Mediums und pro Minute beträgt, und/oder die spezifische Wärmeleistung in das Medium mindestens 0,02 kJ pro kg des flüssigen Mediums pro Minute und pro g/l Trockenmasse der Pflanzenzellen beträgt.

5. Das Verfahren nach einem der Ansprüche 1 bis 4, **dadurch gekennzeichnet, dass** das exprimierte Material Proteine enthält, und/oder dass das exprimierte Material sekretiertes Material ist, vorzugsweise durch die Zellmembran sekretierte Proteine.

6. Das Verfahren nach einem der Ansprüche 1 bis 5, **dadurch gekennzeichnet, dass** der Rotor-Stator in einen Behälter mit dem Medium eingebracht wird.

7. Das Verfahren nach einem der Ansprüche 1 bis 6, **dadurch gekennzeichnet, dass** der Rotor-Stator einen Innenraum aufweist, welcher mindestens je einen Ein- und Ablauf hat, durch die kontinuierlich das flüssige Medium durch den Innenraum befördert wird.

8. Das Verfahren nach einem der Ansprüche 1 bis 7, **dadurch gekennzeichnet, dass** der Stator ein Volumen von 10 cm³ bis 1 m³ begrenzt, und/oder dass die Menge des behandelten flüssigen Mediums bis zu 50 kg ist, vorzugsweise 0,5 g bis 50 kg.

9. Das Verfahren nach einem der Ansprüche 1 bis 8, **dadurch gekennzeichnet, dass** die pflanzlichen Zellen in einer Konzentration von 0,2 g/l bis 60 g/l im flüssigen Medium vorliegen (Masse der Pflanzenzellen als Trockenmasse).

10. Das Verfahren nach einem der Ansprüche 1 bis 9, **dadurch gekennzeichnet, dass** die Pflanzenzellen Mooszellen sind, vorzugsweise *P. patens* Zellen.

11. Das Verfahren nach einem der Ansprüche 1 bis 10, **dadurch gekennzeichnet, dass** der Rotor mit einer Drehzahl von maximal 15000 Umdrehungen pro Minute betrieben wird, vorzugsweise 1000 bis 15000 Umdrehungen pro Minute.

12. Das Verfahren nach einem der Ansprüche 1 bis 11, **dadurch gekennzeichnet, dass** der Rotor-Stator ein Stabhomogenisierer oder eine Scherpumpe ist, und/oder wobei der Stator eine Kammstruktur aufweist.

13. Das Verfahren nach einem der Ansprüche 1 bis 12, **dadurch gekennzeichnet, dass** die durch Rotation des Rotors eingebrachte Wärme des Rotor-Stators maximal 30 kJ pro kg des flüssigen Mediums und die eingebrachte Wärmeleistung in das Medium maximal 1,5 kJ pro kg des flüssigen Mediums und pro Minute beträgt.

14. Das Verfahren nach einem der Ansprüche 1 bis 13 **dadurch gekennzeichnet, dass** das Medium einen pH im Bereich von 5 bis 8 hat und/oder eine Osmolarität von mindestens 0,1 osmol/L hat.

15. Das Verfahren nach einem der Ansprüche 1 bis 14, **dadurch gekennzeichnet, dass** die Pflanzenzellen 2 min bis 150 min mit dem Rotor-Stator behandelt werden.

## Claims

1. A method for detaching expressed material from the surface of or from the apoplast of plant cells, wherein the plant cells are treated with a rotor-stator in a liquid medium, wherein the heat introduced from the rotor-stator by rotation of the rotor is a maximum of 3 kJ per kg of the liquid medium and per g/L dry weight of the plant cells and the heat introduced into the medium is a maximum of 1.5 kJ per kg of the liquid medium per minute and per g/L dry weight of the plant cells.

2. The method as claimed in claim 1, **characterized in that** the expressed material is in the apoplast of the plant cells.

3. The method as claimed in claim 1 or claim 2, **characterized in that** the heat from the rotor-stator introduced by rotation of the rotor is at least 1 kJ per kg of the liquid medium, and/or the heat from the rotor-stator is at least 0.1 kJ per kg of the liquid medium and per g/L of dry weight of the plant cells.

4. The method as claimed in one of claims 1 to 3, **characterized in that** the heat introduced into the medium by rotation of the rotor is at least 0.2 kJ per kg of the liquid medium and per minute, and/or the heat into the medium is at least 0.02 kJ per kg of the liquid medium per minute and per g/L of dry weight of the plant cells.

5. The method as claimed in one of claims 1 to 4, **characterized in that** the expressed material contains proteins, and/or **in that** the expressed material is secreted material, preferably proteins secreted through the cell membrane.

6. The method as claimed in one of claims 1 to 5, **characterized in that** the rotor-stator is introduced into a container with the medium.

7. The method as claimed in one of claims 1 to 6, **characterized in that** the rotor-stator has an interior which has at least one inlet and outlet via which the liquid medium is continuously fed through the interior.

8. The method as claimed in one of claims 1 to 7, **characterized in that** the stator delimits a volume of 10 cm3 to 1 m3, and/or in that the quantity of the treated liquid medium is up to 50 kg, preferably 0.5 g to 50 kg.

9. The method as claimed in one of claims 1 to 8, **characterized in that** the plant cells are in a concentration of 0.2 g/L to 60 g/L in the liquid medium (mass of plant cells as dry weight).

10. The method as claimed in one of claims 1 to 9, **characterized in that** the plant cells are moss cells, preferably *P. patens* cells.

11. The method as claimed in one of claims 1 to 10, **characterized in that** the rotor is operated at a maximum rotational speed of 15,000 revolutions per minute, preferably 1,000 to 15,000 revolutions per minute.

12. The method as claimed in one of claims 1 to 11, **characterized in that** the rotor-stator is a rod homogenizer or a shear pump, and/or wherein the stator has a comb structure.

13. The method as claimed in one of claims 1 to 12, **characterized in that** the heat from the rotor-stator introduced by rotation of the rotor is a maximum of 30 kJ per kg of the liquid medium and the heat introduced into the medium is a maximum of 1.5 kJ per kg of the liquid medium and per minute.

14. The method as claimed in one of claims 1 to 13, **characterized in that** the medium has a pH in the range 5 to 8 and/or an osmolarity of at least 0.1 osmol/L.

15. The method as claimed in one of claims 1 to 14, **characterized in that** the plant cells are treated with the rotor-stator for 2 min to 150 min.

## Revendications

1. Procédé de détachement de matériel exprimé de la surface ou de l'apoplaste de cellules végétales, dans lequel les cellules végétales sont traitées dans un milieu liquide avec un rotor-stator, dans lequel la chaleur spécifique du rotor-stator introduite par la rotation du rotor est au maximum de 3 kJ par kg du milieu liquide et par g/l de matière sèche des cellules végétales, et la puissance thermique spécifique introduite dans le milieu est au maximum de 1,5 kJ par kg du milieu liquide par minute et par g/l de matière sèche des cellules végétales.

2. Le procédé selon la revendication 1, **caractérisé en ce que** le matériel exprimé se trouve dans l'apoplaste des cellules végétales.

3. Le procédé selon la revendication 1 ou 2, **caractérisé en ce que** la chaleur du rotor-stator introduite par la rotation du rotor est d'au moins 1 kJ par kg du milieu liquide, et/ou la chaleur spécifique du rotor-stator est d'au moins 0,1 kJ par kg du milieu liquide et par g/l de matière sèche des cellules végétales.

4. Le procédé selon l'une des revendications 1 à 3, **caractérisé en ce que** la puissance thermique introduite dans le milieu par la rotation du rotor est d'au moins 0,2 kJ par kg du milieu liquide et par minute, et/ou la puissance thermique spécifique introduite dans le milieu est d'au moins 0,02 kJ par kg du milieu liquide par minute et par g/l de matière sèche des cellules végétales.

5. Le procédé selon l'une des revendications 1 à 4, **caractérisé en ce que** le matériel exprimé contient des protéines, et/ou **en ce que** le matériel exprimé est du matériel sécrété, de préférence des protéines sécrétées à travers la membrane cellulaire.

6. Le procédé selon l'une des revendications 1 à 5, **caractérisé en ce que** le rotor-stator est introduit dans un récipient contenant le milieu.

7. Le procédé selon l'une des revendications 1 à 6, **caractérisé en ce que** le rotor-stator présente un espace intérieur qui comporte au moins une entrée et une sortie, à travers lesquelles le milieu liquide est transporté en continu à travers l'espace intérieur.

8. Le procédé selon l'une des revendications 1 à 7, **caractérisé en ce que** le stator délimite un volume de 10 cm³ à 1 m³, et/ou en ce que la quantité de milieu liquide traité est jusqu'à 50 kg, de préférence de 0,5 g à 50 kg.

9. Le procédé selon l'une des revendications 1 à 8, **caractérisé en ce que** les cellules végétales sont présentes dans le milieu liquide à une concentration de 0,2 g/l à 60 g/l (masse des cellules végétales en matière sèche).

10. Le procédé selon l'une des revendications 1 à 9, **caractérisé en ce que** les cellules végétales sont des cellules de mousse, de préférence des cellules de *P. patens.*

11. Le procédé selon l'une des revendications 1 à 10, **caractérisé en ce que** le rotor est actionné à une vitesse de rotation maximale de 15000 tours par minute, de préférence de 1000 à 15000 tours par minute.

12. Le procédé selon l'une des revendications 1 à 11, **caractérisé en ce que** le rotor-stator est un homogénéisateur à tige ou une pompe de cisaillement, et/ou dans lequel le stator présente une structure en peigne.

13. Le procédé selon l'une des revendications 1 à 12, **caractérisé en ce que** la chaleur du rotor-stator introduite par la rotation du rotor est au maximum de 30 kJ par kg du milieu liquide et la puissance thermique introduite dans le milieu est au maximum de 1,5 kJ par kg du milieu liquide et par minute.

14. Le procédé selon l'une des revendications 1 à 13, **caractérisé en ce que** le milieu a un pH compris entre 5 et 8 et/ou une osmolarité d'au moins 0,1 osmol/L.

15. Procédé selon l'une des revendications 1 à 14, **caractérisé en ce que** les cellules végétales sont traitées avec le rotor-stator pendant 2 min à 150 min.
